# EUROPEAN PATENT SPECIFICATION

(11) **EP 2 209 793 B1**
(45) Date of publication and mention of the grant of the patent: **21.08.2013**
(21) Application number: 08839703.9
(22) Date of filing: 02.10.2008
(51) Int. Cl.: C07F 5/02, C07C 311/24, C07C 41/09, C07C 201/08, C07C 309/06, B01J 31/02

(54) **SUPPORTED IONIC LIQUIDS OR ACTIVATING SUPPORTS**
GESTÜTZTE IONISCHE FLÜSSIGKEITEN ODER ACTIVIERENDE TRÄGER
LIQUIDES IONIQUES SUPPORTÉS OU SUPPORTS ACTIVANTS

(30) Priority: 19.10.2007 EP 07291284
(43) Date of publication of application: 28.07.2010
(73) Proprietor: TOTAL RESEARCH & TECHNOLOGY FELUY, 7181 Seneffe (BE); Centre National de la Recherche Scientifique (CNRS), 75016 Paris (FR)
(72) Inventor: EL KADIB, Abdelkrim, F-34000 Montpellier (FR); MOLVINGER, Karine, F-34080 Montpellier (FR); BRUNEL, Daniel, F-34090 Montpellier (FR); PRADES, Floran, B-1400 Nivelles (BE); RAZAVI, Abbas, B-7000 Mons (BE)
(74) Representative: Leyder, Francis
(86) International application number: PCT/EP2008/063211
(87) International publication number: WO 2009/050042

(56) References cited:
- WO-A-01/32308
- WO-A-93/11172
- WO-A-02/098560
- US-B1- 6 395 673
- DATABASE CA [Online] CHEMICAL ABSTRACTS SERVICE, COLUMBUS, OHIO, US; NISHIKIDO, JOJI ET AL: "Acid catalyst fixed on a metal oxide support for aqueous reactions" XP002473314 retrieved from STN Database accession no. 2005:344155

## Description

The present invention relates to the covalent anchorage of non-coordinating anions on mineral supports to prepare supported ionic liquids and to their use as activating agents for the polymerisation of ethylene and alpha-olefins.

It is known that perfluorinated borates can be covalently anchored on different supports such as for example polystyrene and silica. This was reported in US-A-5427991 or in US-A-5869723 or in EP-A-1359166 or in WO03/035708. The counter cations of the grafted perfluorinated borate were anilinium-type or trityl cations when these solid systems were used to activate catalyst components in the polymerisation of ethylene and alpha-olefins. In parallel, various works were reported in literature concerning the direct chemisorption of tris (pentafluorophenyl)borane on silica surface such as for example J. Tian, S. Wang, Y. Feng, J. Li, S. Collins, "Borane-functionalized oxide supports: development of active supported metallocene catalysts at low aluminoxane loading" in J. Mol. Catal. A: Chemical , 1999, 144, 137-150, or in M. Bochmann, G.J. Pindado, S.J. Lancaster, "The versatile chemistry of metallocene polymerisation catalysts: new developments in half-sandwich complexes and catalyst heterogenisation." in J. Mol. Catal. A: Chemical, 1999, 146, 179-190, or in S. Charoenchaidet, S. Chavadej, E. Gulari, "Borane-functionalized silica supports in situ activated heterogeneous zirconocene catalysts for MAO-free ethylene polymerisation" in J. Mol. Catal. A: Chemical, 2002, 167-177, 185, or in K. Musikabhumma, T.P. Spaniol, J. Okuda, "Ethylene polymerization with "constrained-geometry" titanium catalysts over borate-modified silica supports" in Macromol. Chem. Phys., 2002, 203, 115-121. This strategy additionally requires the presence of trityl or substituted ammonium (preferably dimethylanilinium) as countercations.

These catalytic systems using supported activators are less active than equivalent homogeneous systems and the polymer properties are thereby degraded.

A new generation of solid activating supports has been developed and is described for example in Marks (J. Am. Chem. Soc., 1998, 120, 13533): it concerns sulfated zircone particles or in McDaniel (WO-9960033, WO-0123433, WO-0123434, WO-0144309, WO-0149747 et US-A-6548441) or in Saudemont (FR-A-2765225). All these activators are solids wherein surface acid sites are responsible for the activation.

These acid sites are metals combined with halides such as fluor or chlorine; metals can be selected from aluminium, titanium, zirconium or nickel.

The equivalent species in homogeneous catalysis are very poor activating species.

Compounds such as dimethylaluminium fluoride (DMAF) are used as activators in combination with triethylaluminium for the stereospecific polymerisation of propylene with compounds of the metallocene family with low productivity as described by Zambelli (Macromolecules 1989, 22, 2186). They do not activate metallocene complexes.

On the other hand, ionic liquids are also actively investigated as alternative solvent media in many industrial applications such as synthesis, catalysis, separation, electrochemistry or nanoparticles stabilisation ( *"*Ionic Liquids -Industrial Applications to Green Chemistry" ACS Symposium Series, Eds RD. Rogers and K.S. Seddon, American Chemical Society, Washington, DC, 2001 ; "Innovative Applications of Ionic Liquids as "Green Engineering Liquids" H. Zhao, Chem. Eng. Comm., 2006, 193, 1660-1677). Synthesis is described for example in K.R. Seedon, Kinetic Catal., 1996, 37, 693; or in J. Howarth, Tetrahedron Lett., 2000, 41, 6627; or in F. Zulfiquar, G. Tanaka, Green Chemistry, 2000, 2, 6627. Catalysis is described for example in "Catalytic reactions in ionic liquids" R. Sheldon, Chem. Commun., 2001, 2399-2407; or in J.J. Peng, Y.Q. Deng, New J. Chem.*,* or in *"*Ionic liquids in Catalysis"T. Welton, Coord. Chem. Rev., 2004, 248, 2459-2477. Separation is described for example in H. Qiu, S. Jiang, X. Liu, J. Chromatography A, 2006, 1103, 265-270; or in H. Qiu, S. Jiang, X. Liu, L. Zhao, J. Chromatography A, 2006, 1106, 46-50. Electrochemistry is described for example in J. Dupont, R.F. de Souza, P.A.Z. Suarez, Chem. Rev., 2002, 102, 3667-369. Nanoparticle stabilisation is described for example in "The use of imidazolium ionic liquids for the formation and stabilization of Ir0 and Rh0 nanoparticles: efficient catalysts for the hydrogenation of arenes" G.S. Fonseca, A.P. Umpierre, P.F.P. Fichtner, S. Teixeira, Chem. Eur.J. 2003, 9, 3263-3269.

Perfluoroalkyltrifluoroborates were also used as counter anions for ionic liquids, particularly to stabilise cyclic quaternary ammonium as disclosed for example in "Cyclic quaternary ammonium ionic liquids with perfluoroalkyltrifluoroborates: synthesis, characterization, and properties" Z.-B. Zhou, H. Matsumoto, K. Tatsumi, Chem. Eur. J., 2006, 12, 2196-2212. Until now, ionic liquids have been covalently anchored on silica surface by means of their cationic part using silica silylating reagent such as 1-methyl-3-(3-triethoxysilyl)-propylimidazolium cation as disclosed for example in WO2002098560, or in WO2001032308, or in M.H. Valkenberg, C. de Castro, W.F. Höelderich, Green Chemistry, 2002, 4, 88-93, or in C.P. Mehnert, R.A. Cook, N.C. Dispenziere, M. Afework, J. Am. Chem; Soc., 2002, 124, 12932.

The ionic liquids can also be anchored by their anionic part: this is achieved by codeposition of chloroaluminate species anion on silica with the 1-butyl-3-methylimidazolium (bmim) as counter cation.

N-[(trifluoromethyl)sulfony] trifluoromethanesulfonimide or bis-((perfluoromethyl)sulfonyl)imide (TFMSI) is currently used as counter anion in ionic liquids in order to impart to the resulting ionic liquid, particular physico-chemical properties. This is described for example in "Synthesis of 2,2-biimidazolium-based ionic liquids: use as a new reaction medium and ligand for palladium-catalyzed Suzuki cross-coupling reactions" J-C. Xiao, J.M. Shreeve, in J. Org. Chem., 2005, 70, 3072-3078, or in "Lithium ion conduction in a organoborate zwitterion-LiTFSI mixture" A. Narita, W. Shibayama, K. Sakamoto, T. Mizumo, N. Matsumi, H. Ohno, in Chem. Commun, 2006, 1926-1928.

One of the objectives of this invention is the anchorage of two parts of ionic liquids onto a support: the anion and the cation.

It is a further goal of the present invention to prepare supported ionic liquids from N[(perfluoroalkyl)sulfonyl] perfluoroalkane sulfonimide (PFSI) anions covalently anchored on mineral support and to use them as activating supports for the polymerisation of ethylene and alpha-olefins.

It is an aim of the present invention to prepare new species of activating support.

It is another aim of the present invention to prepare very active single site supported catalyst systems that do not require the use of methylaluminoxane.

It is also an aim of the present invention to prepare polymers that have regular grain size.

Any one of these aims is at least partially fulfilled by the present invention.

The present invention relates to the covalent anchorage of non-coordinative perfluorinated anions on mineral oxide support.

Accordingly, the present invention discloses the preparation of covalently anchored N[(perfluoroalkyl)sulfonyl]perfluoroalkanesulfonamide system as precursors for the preparation of supported ionic liquids that can be used as activating supports suitable for the polymerisation of ethylene and alpha-olefins.

Perfluoroalkanesulfonic acid-containing solids, generally called "Nafion"type-functionalised silica, have been previously reported by Harmer et al. ("Unique silane modified perfluorosulfonic acids as versatile reagents for new solid acid catalysts" M.A. Harmer, Q. Sun, M.J. Michalczyk, Z. Yang, in Chem. Commun., 1997, 1803-1804; or in "Novel mesoporous silica-perfluorosulfonic acid hybrids as strong heterogeneous Brönsted catalysts" D.J. Macquarrie, S.J. Taverner, M.A. Harmer, in Chem. Commun., 2005, 263-265) and by Corma et al. ( "Single-step preparation and catalytic activity of mesoporous MCM-41 and SBA-15 silicas functionalized with perfluorosulfonic acid groups analogous to Nafion " M. Alvaro, A. Corma, D. Das, V. Fornes, H. Garcia, in Chem. Commun., 2004, 956-957; or in "Nafion" functionalized mesoporous MCM-41 silica shows high activity and selectivity for carboxylic acid esterification and Friedel-Crafts acylation reactions, M. Alvaro, A. Corma, D. Das, V. Fomes, H. Garcia, J. Catal., 2005, 231, 48-55; or in WO 2005051540). They were used in supported acid catalysis such as electrophilic aromatic substitution reactions and fatty acid esterification.

Corma's supported perfluorosulfonic acid is the reaction product of silica surface on trifluoromethyl-1 trifluoro 1,2,2'- ethanesultone, said latter product being the reaction product of perfluoropropene and sulphur trioxide. Alternatively supported sulfonic acid can be obtained starting from perfluorinated alkan-1ene.

There are two types of supported perfluorinated sulfonic acids respectively represented in the schemes below as starting material 1 and starting material 2.

The present invention discloses a method for preparing supported ionic liquids that comprises the steps of:
a) reacting starting supported sulfonic acid 1 or 2 either with non-nucleophilic trimethylsilane in scheme (a) or with perfluroalkan-2-ethyltrialkoxysilane in scheme b) in order to make the sulfonic group environment hydrophobic 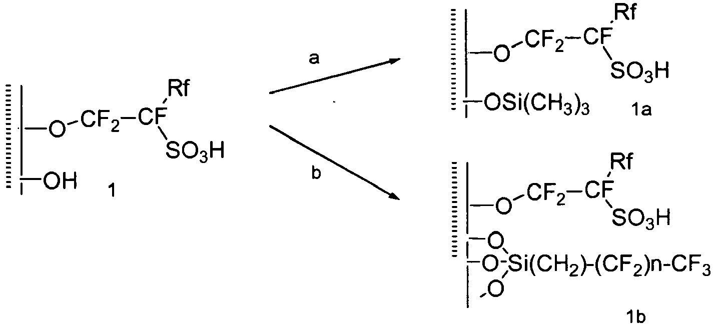 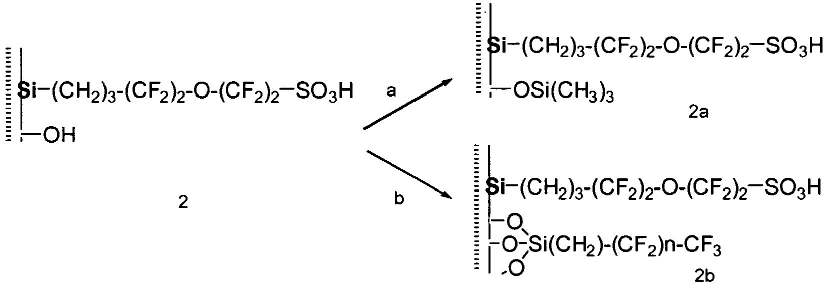 wherein Rf is perfluoroalkyl group CF₃(CF₂)ₙ- or (CF₃)₂CF(CF₂)ₙ- and wherein n is 0 or integer from 1 to 8;
b) reacting compounds 1, 1a, 1b, 2a and 2b with thionylchloride to produce respectively supported perfluorosulfonic chloride products 1c, 1d, 1e, 2d and 2e 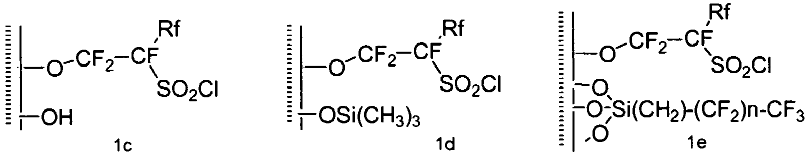 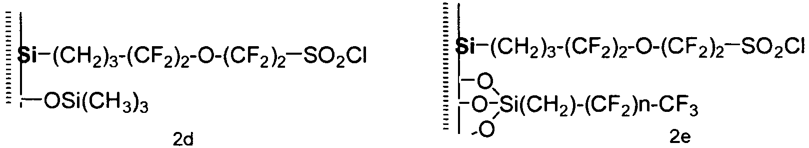
c) reacting perfluorosulfonyl chloride group-containing solids 1c, 1d, 1e, 2d, and 2e with ammonia to prepare perfluorosulfonamides compounds 1f, 1g, 1h, 2g and 2h 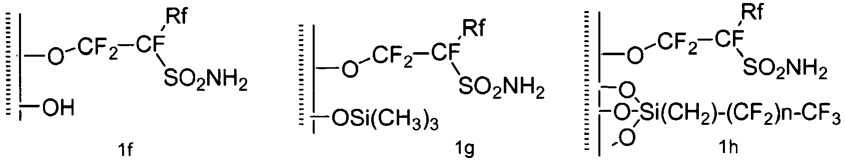 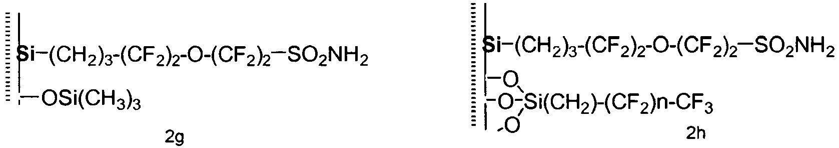
d) reacting perfluorosulfonamides compounds 1f, 1g, 1h, 2g and 2h with perfluoroalkanesulfonyl chloride Rf'SO₂Cl in the presence of an organic base to prepare respectively the covalently anchored N[(perfluoroalkyl)sulfonyl] perfluoroalkane sulfonamide 1i, 1j, 1 k, 2j and 2k 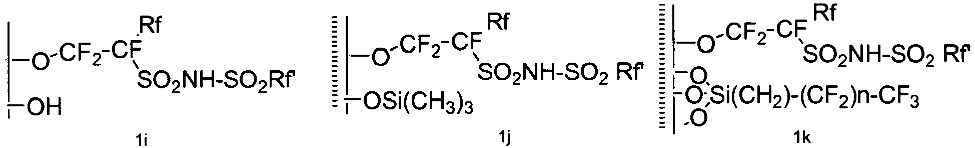 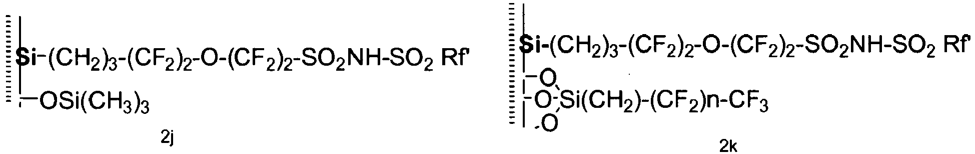 wherein Rf is perfluoroalkyl group CF₃(CF₂)ₙ- or (CF₃)₂CF(CF₂)ₙ-, wherein n is 0 or an integer from 1 to 8 and wherein Rf is the same as or different from Rf.
e) reacting anchored N[(perfluoroalkyl)sulfonyl) perfluoroalkane sulfonamide 1i, 1j, 1 k, 2j and 2k with a metal hydroxide MOH or a metal organic derivative such as alkylate, arylate, aryl hydride or an organic base such as M-isopropyl amide or M-hexamethyldisilylazide, wherein M is a metal Group 1 of the periodic table, in order to obtain the corresponding covalently anchored N[(perfluoroalkyl)sulfonyl]⁻ perfluoroalkane sulfonimide salts 1i', 1j', 1 k', 2j' and 2k' 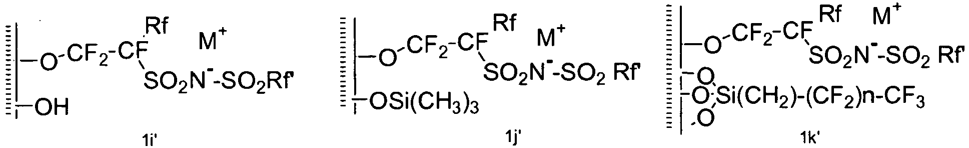 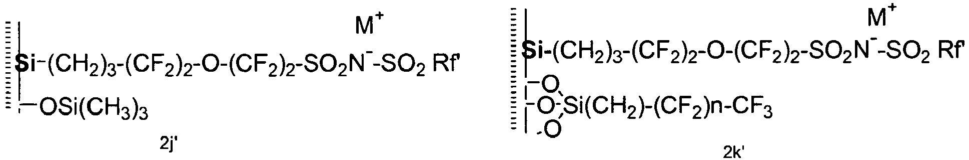
f) reacting the N[(perfluoroalkyl)sulfonylrperfluoroalkane sulfonimide salts-anchored supports of step e) with an organo-cation Cat, in order to obtain respectively compounds 1l, 1m, 1 n, 2m and 2n 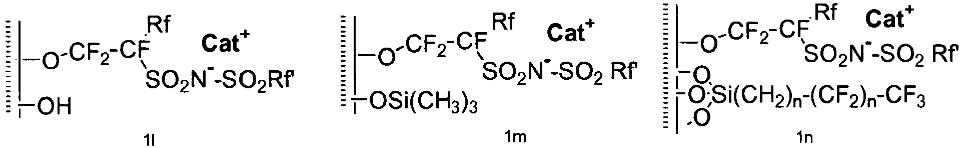 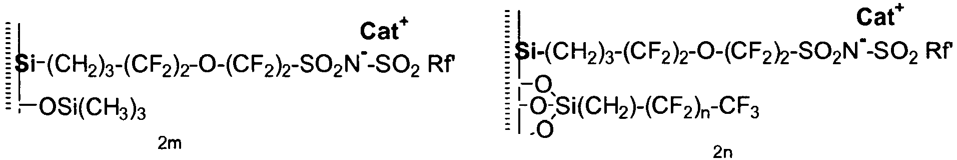 wherein Cat is trityl Ph₃CX or ammonium halide R₁R₂R₃R₄N⁺X⁻, wherein X is halogen, Ph is phenyl group, and R₁, R₂, R₃ and R₄ are each independently selected from hydrocarbons having from 1 to 6 carbon atoms.

Preferably at least one of R₁, R₂, R₃ and R₄ is aromatic group, more preferably phenyl group.

Preferably, M is selected from Li, Na, K. More preferably it is Li. The preferred metal organic derivative is lithium hexamethyldisilylazide: it offers the advantage of passivating the residual silanols by trimethylsilylation.

The preferred organic base of step d) is triethylamine.

Preferably, Rf and Rf' are the same. More preferably they both are CF₃.

All supported ionic liquids according to the present invention can be used as activating supports, but not all activating supports are ionic liquids.
- When the cation is selected from trityl or aniline derivatives and the counter-anion is selected from halide or carboxylate, sulfonate, the resulting supported complex is an activating support suitable for the polymerisation of ethylene and alpha-olefins.
- When the cation is selected from dialkylimidazolium or 1-alkyl-pyridinium and related systems, the resulting complex is a supported ionic liquid.

In a preferred embodiment according to the present invention N-alkyl-N'-3-(propyltrialkoxysilyl) imidazolium is used as counter cation for co-grafting the cationic part of the ionic liquid system and PFSI is used as anionic part.

In another embodiment according to the present invention, N[(perfluoroalkyl)sulfonyl] perfluoroalkane sulfonimide compounds 1 i, 1j, 1 k, 2j and 2k are synthesised in one step by reacting the perfluorosulfonyl chloride group-containing solids 1c, 1d, 1e, 2d and 2e of step b) with perflorosulfonamide Rf'SO₂NH₂ in presence of a solution of organic base.

In yet another embodiment according to the present invention, related to the supported perfluorosulfonic compounds of starting material 1, the reaction of step f), between compound 1i' and an organo-cation, is replaced by addition to compound 1i' of 1,3-diakyl-imidazolium bearing trialkoxysilyl groups (imid), that reacts with the residual hydroxyl groups of the mineral oxide surface resulting in anchorage of both the anionic and cationic parts to the support 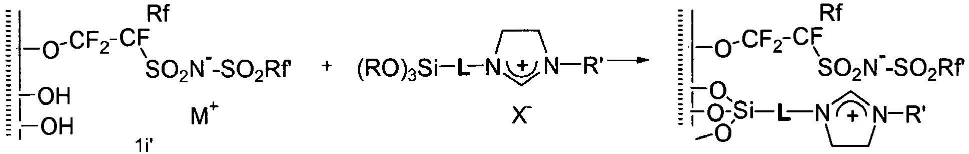 wherein linker group L is alkane or aryl group, R' is alkyl or aryl group, functionalised or not, and Rf and R'f are the same or different and are perfluoroalkyl groups CF₃(CF₂)ₙ- or (CF₃)₂CF(CF₂)ₙ- wherein n is zero or an integer from 1 to 8.

The support is preferably a silica support.

The present invention also discloses the activating supports and the supported ionic liquids obtainable by the method of the present invention.

These new supported ionic liquids and activating supports are suitable for preparing catalyst systems based on single site catalyst components. Metallocene or late transition metal complex catalyst components prepared by any method known in the art can be deposited on the supported ionic liquids or activating supports of the present invention in order to provide active catalyst systems without addition of conventional activating agents such as aluminoxanes or boron-based compounds. The metallocene components that are preferably used in the present invention are fully alkylated and are based on bridged bisindenyl, bistetrahydroindenyl and cyclopentadienyl-fluorenyl. The most preferred catalyst components are selected from ethylene bis tetrahydroindenyl zirconium dimethyl, ethylene bis(2-Me,4-Ph-indenyl) zirconium dimethyl and ethylene (2-Me,4-tBut-cyclopentadienyl)(3,6-tBut-fluorenyl) zirconium dimethyl. Late transition metal complexes of the present invention preferably include α-diimine Ni complexes as disclosed by Brookhart in W096/ 23010 or bis(imino)pyridyl Iron(II) or Cobalt(II) complexes as disclosed by Bristovsek et al. (G.J.P. Bristovsek, V.C. Gibson, B.S. Kimberley, P.J. Maddox, S.J. Mc Tavish, G.A. Solan, A.J.P. White, D.J.J. Williams, in Chem. Common., 849-50, 1998) or in Small et al. (B.L. Small, M. Brookhart, A.M.A. Bennett, in J. Am. Chem. Soc., 120, 4049, 1998).

The metallocene component is preferably fully dialkylated. If it is dihalogenated, it is preferably used with an alkylating agent such as for example aluminium alkyl.

The present invention thus discloses a method for preparing an active catalyst system by the steps of:
a) providing a supported ionic liquid or an activating support as described in any one of the embodiments hereabove;
b) impregnating a fully alkylated metallocene or a post-metallocene catalyst component onto the activating support or supported ionic liquid;
c) optionally adding a scavenger;
d) retrieving an active catalyst system.

The scavenger is preferably an alkyl aluminium. More preferably, it is trimethyl aluminium (TMA), triethyl aluminium (TEAL) or triisobutyl aluminium (TIBAL).

The active catalyst systems of the present invention are used for the oligomerisation or the homo- or co-polymerisation of ethylene and alpha-olefins. They have the advantage of being very efficient without the need to add costly and dangerous material such as methylaluminoxane.

The present invention thus discloses a method for oligomerising or homo- or co-polymerising that comprises the steps of:
a) injecting the active catalyst system of the present invention into the reactor;
b) injecting the monomer and optional comonomer simultaneously with or after the catalyst system;
c) optionally injecting a scavenger;
d) maintaining under polymerization conditions;
e) retrieving a polymer or oligomer.

The monomer is preferably ethylene or propylene. The comonomer is preferably ethylene, propylene or 1-hexene.

### Examples.

The starting silica support used in all examples was G5H from Grace Davison with a surface area of 513 m².g⁻¹ and a porous volume of 1.8 mL.g⁻¹.

### Preparation of 1,2,2-trifluoro-1-trifluoromethyl-ethane sulfonic acid-containing silica gel.

### Sample I, precursor formula 1.

A solution of 0.8 g (3.5 mmol) of 1,2,2-trifluoro-2-hydroxy1-(trifluoromethyl)-ethane sulfonic acid beta-sultone (from ABCR chemicals) in dry toluene (20mL) was added at room temperature to a suspension of 4 g of cooled activated silica gel ((G5H) (treated under vacuum at a temperature 150°C during 8h ) in toluene (25mL) under argon. After stirring at room temperature during 30 min, the reaction mixture was heated at a temperature of 80°C during 12h then at a temperature of 100°C during 3h more. After cooling, the solid was filtered under argon and washed under argon with dry toluene (30mL, 2 times), then with pentane (20mL) then evacuated under vacuum 4h. Thermogravimetry analysis indicated a perfluorosulfonic loading of 0.2 mmol.g⁻¹

### Sample II.

Two other runs were carried out. In each run, 2.8 g of sultone (12 mmol) were contacted with 6g of activated silica using the same procedure. Thermogravimetry analysis indicated a perfluorosulfonic loading of 0.54 mmol.g⁻¹.

### Passivation of samples I and II.

3.2 g of perfluorosulfonic acid -grafted silica of samples I and II were evacuated at a temperature of 100°C during 12h then each sample was treated with a solution of *N,O*-bis-trimethylsilyl-trifluoroacetamide (2 mL) in toluene (32 mL) and stirred. Each reaction mixture was heated at a temperature of 80°C during 12h. The passivated perfluorosulfonic acid -grafted silicas were filtered under argon, then washed with toluene (2 times 20 mL), pentane (2 times ,20mL) dichloromethane (2 times 20mL), then ethyl-ether (20 mL). The two passivated solids were then evacuated under vacuum.

### Preparation of passivated 1,2,2-trifluoro-1-trifluoromethyl-ethane sulfonyl chloride-containing silica gel.

3.2 g of each of the passivated sulfonic acid-grafted silica samples prepared hereabove were activated at a temperature of 100°C under vacuum during 12h. After cooling, 40mL of dry toluene were added under argon, followed by addition of freshly distilled SOCl₂ (4mL), corresponding to a large excess (4 equ.) of grafted sulfonic function. After stirring at room temperature during 30 min, the reaction mixtures were heated at a temperature of 110°C during 2h. After cooling, the excess SOCl₂ was evacuated under vacuum.

### Preparation of N-[(trifluoromethyl)sulfonyl-(1,2,2-trifluoro-1-trifluoromethyl-ethane) sulfonamide-containing silica gel.

### First method: Preparation of passivated 1,2,2-trifluoro-1-trifluoromethyl-ethane sulfonamide-containing silica gel

3g of the passivated 1,2,2-trifluoro-1-trifluoromethyl-ethane sulfonyl chloride-containing silica gel obtained hereabove were poured in an autoclave (50mL) under a stream of argon. After cooling the autoclave at a temperature of -15°C, ammonia was admitted (5.5 bars) until the ammonia condensed weight was 6 g. The autoclave was gently returned to room temperature, the reaction mixture was maintained under stirring forr 3h30 at room temperature. Ammonia was then evacuated at normal pressure. The solid was washed with toluene (3 times , 30 mL), ethanol (30mL), ethanol-water mixture (2 times, 30mL), ethanol (20mL), filtered, then washed again with ethanol (20mL), pentane (2 times, 10 mL), then evacuated under vacuum.

### Preparation of passivated N-[(trifluoromethyl)sulfonyl]-(1,2,2-trifluoro-1-trifluoromethyl-ethane) sulfonamide-containing silica gel.

To a suspension of passivated 1,2,2-trifluoro-1-trifluoromethyl-ethane sulfonamide-containing silica gel (1.5 g), previously activated at a temperature of 120°C during 6 h, then cooled under argon, in toluene (20mL), was added a solution of trifluoromethanesufonyl chloride (0.68g ; 4 mmol) and triethylamine (0.4 g; 4 mmol) in toluene (15 mL).

The reaction mixture was stirred at a temperature of 80°C during 12h, then after cooling the solid was filtered , washed with toluene, methanol : water mixture (60:40), ethanol, ether, then with a refluxing dichloromethane: ethyl ether mixture (50:50) using a soxhlet apparatus. The solid was washed twice with pentane (30 mL) and dried under vacuum.

### Second method: Preparation of N-[(trifluoromethyl)sulfonyl]-(1,2,2-trifluoro-1-trifluoromethyl-ethane) sulfonamide-containing silica gel.

1,2,2-trifluoro-1-trifluoromethyl-ethane sulfonyl chloride-containing silica gel (1.6 g) was freshly prepared from 1,2,2-trifluoro-1-trifluoromethyl-ethane sulfonic acid-containing silica gel (sample III). It was prepared following the same procedure as that used to prepare sample II and contained ∼1 mmol.g⁻¹: it was obtained by successive treatment with 4 mL of toluene and of SOCl₂ (3 mL, 25 mmol), at 100°C during 6h, then evacuation under vacuum during 4h. After cooling, the solid was suspended in toluene (25 mL) under argon and trifluoromethylsulfonamide (1g, 6.7 mmol, corresponding to 5 eq. vs sulfonyl chloride loading) was poured into the reaction mixture, then triethylamine ( 0.8 mL, corresponding to ∼5eq vs sulfonyl chloride loading) was injected with a syringe. The reaction mixture was heated at 90°C under stirring during 16h. After cooling, the solid was filtered, washed with toluene (50 mL, 3 times), washed with toluene, methanol : water mixture (60:40), ethanol, ether, then with a refluxing dichloromethane: ethyl ether mixture (50:50) using a soxhlet apparatus. The solid was washed twice with pentane (30 mL) and dried under vacuum.

### Preparation of trityl N-[(trifluoromethyl)sulfonyl]-(1,2,2-trifluoro-1-trifluoro-methyl-ethane) sulfonimide-containing silica gel.

In a first step, lithium hexamethyldisilazide was prepared by reaction of BuLi (2.5 mL, 0.3 10⁻² mol) in anhydrous THF (35 mL) with hexamethyldisilazane (0.65 mL, 0.3 10⁻² mol) at a temperature of -70°C under argon. The temperature was gently increased to -30°C. The solution was then poured dropwise into a stirred suspension of freshly activated N-[(trifluoromethyl)sulfonyl]-(1,2,2-trifluoro-1-trifluoromethyl-ethane) sulfonamide-containing silica gel (1.5 g; 0.3 10⁻³ mol of sulfonamide) in anhydrous THF (35 mL) at a temperature of 0°C. Then the temperature was allowed to return gently to room temperature and the reaction mixture was stirred during 1 h.

Then, a solution of trityl chloride (0.32 g, 1.2 mmol) in THF (20 mL) was added to the reaction mixture that was stirred at room temperature during 16h. The solid so obtained was filtered, under argon, then washed with anhydrous THF (twice with 30 mL), toluene (twice with 30mL) then with pentane (twice with 30mL), then evacuated under vacuum at room temperature during 4h.

The same procedure was applied to the passivated N-[(trifluoromethyl)sulfonyl]-(1,2,2-trifluoro-1-trifluoromethyl-ethane) sulfonamide-containing silica gel in order to prepare the passivated trityl N-[(trifluoromethyl)sulfonyl]-(1,2,2-trifluoro-1-trifluoromethyl-ethane) sulfonimide-containing silica.

### Preparation of zwitterionic 1-N-methyl-(3-silylpropyl)-3-N'-imidazolium.

### N-[(trifluoromethyl)sulfonyl]-(1,2,2-trifluoro-1-trifluoro-methyl-ethane) sulfonimide-containing silica gel.

A sample of non-passivated lithium N-[(trifluoromethyl)sulfonyl]-(1,2,2-trifluoro-1-trifluoromethyl-ethane) sulfonimide-containing silica (1.7 g) was suspended in THF (20 mL). Then a solution 1-methyl-3-(3-triethoxysilyl)-propylimidazolium chloride ( 0.35 g, 1.12 mmol) was poured under argon into the suspension. The reaction mixture was refluxed for 16h to undergo a condensation reaction of the silylated imidazolium derivatives on the residual silanols. The ionic liquid -supported silica was then filtered, then washed with (20 mL, twice), methanol : eau (2:1) mixture (20 mL, twice) in order to dissolve the lithium choride salts, then with methanol 100%, then with a mixture of dichloromethane : diethyl ether in a Soxhlet apparatus. The solid was washed twice with pentane (30 mL) and dried under vacuum.

### Poymerisation of ethylene.

The supported ionic liquids and activating supports prepared in the examples hereabove were used to polymerise ethylene under the following conditions:
Solvent: 20 mL of heptane
Scavenger: 1 mL of triisobutylaluminium (TIBAL)
Pressure: 15 bars of ethylene
Temperature: 50°C
Polymerisation time: 30 minutes
Stirring was carried out at 1000 rpm
Metallocene catalyst component: a fresh solution of a dimethylated metallocene catalyst component sold by Total Petrochemicals under the name Z12Hsm®.
Activating support : 10 mg of 1,2,2-trifluoro-1-trifluoromethyl-ethane sulfonic acid-containing silica gel, passivated.
Ratio metallocene / activating support: 1.08.

Polyethylene was obtained with a productivity of 63 g/g/h and an activity of 0.34 kg/mmol/h. The morphology was good.

## Claims

1. A method for preparing supported ionic liquids or activating supports by covalent anchorage of non-coordinative perfluorinated anions on mineral oxide support that comprises the steps of:
a) reacting starting supported sulfonic acid 1 or 2 either with non-nucleophilic trimethylsilane in scheme (a) or with perfluroalkan-2-ethyltrialkoxysilane in scheme b) in order to make the sulfonic group environment hydrophobic 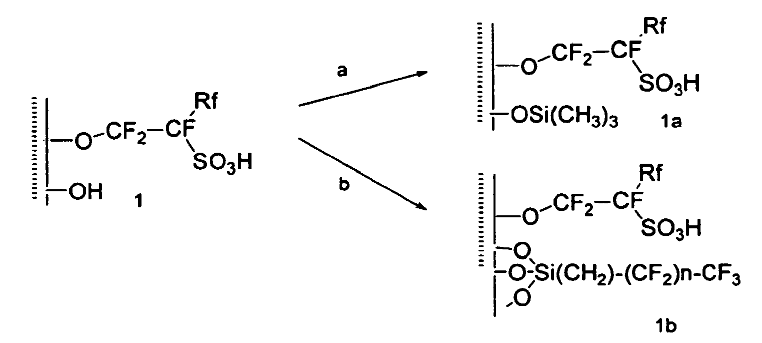 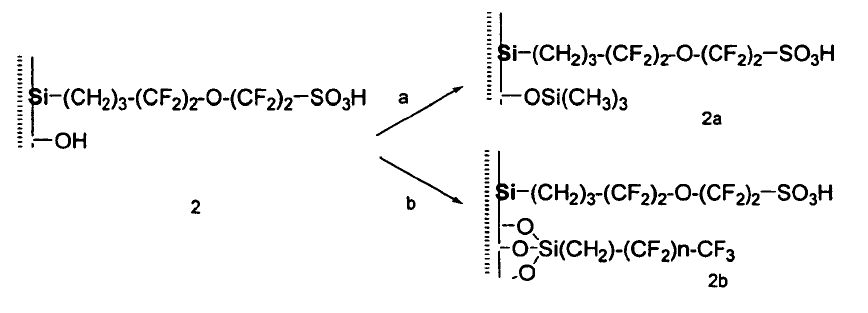 wherein Rf is perfluoroalkyl group CF₃(CF₂)ₙ- or (CF₃)₂CF(CF₂)ₙ-and wherein n is 0 or integer from 1 to 8;
b) reacting compounds 1, 1a, 1b, 2a and 2b with thionylchloride to produce respectively supported perfluorosulfonic chloride products 1c, 1d, 1e, 2d and 2e 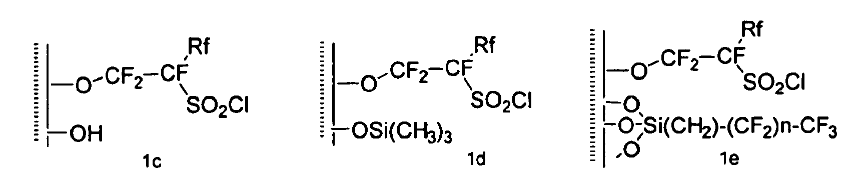 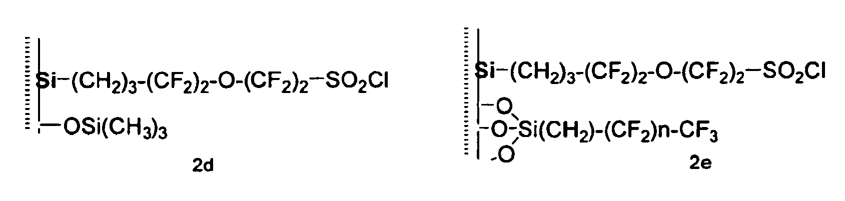
c) reacting perfluorosulfonyl chloride group-containing solids 1c, 1d, 1e, 2d, and 2e with ammonia to prepare perfluorosulfonamides compounds 1f, 1g, 1h, 2g and 2h 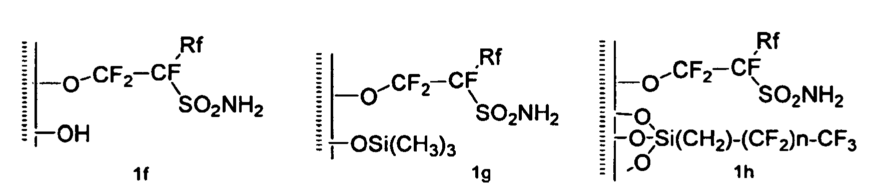 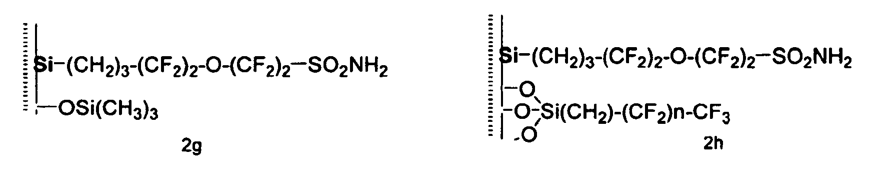
d) reacting perfluorosulfonamides compounds 1f, 1g, 1h, 2g and 2h with perfluoroalkanesulfonyl chloride Rf'SO₂Cl in the presence of an organic base to prepare respectively the covalently anchored N[(perfluoroalkyl)sulfonyl] perfluoroalkane sulfonamide 1i, 1j, 1k, 2j and 2k 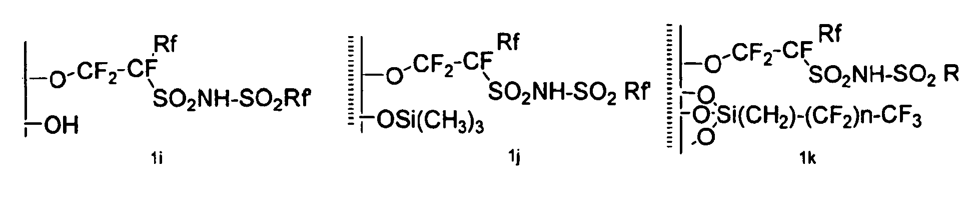 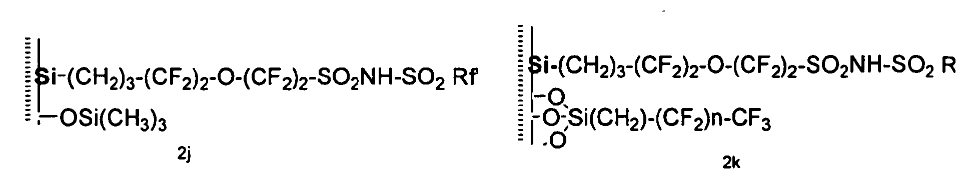 wherein Rf is perfluoroalkyl group CF₃(CF₂)ₙ- or (CF₃)₂CF(CF₂)ₙ-, wherein n is 0 or an integer from 1 to 8 and wherein Rf is the same as or different from Rf.
e) reacting anchored N[(perfluoroalkyl)sulfonyl] perfluoroalkane sulfonamide 1i, 1j, 1k, 2j and 2k with a metal hydroxide MOH or a metal organic derivative such as alkylate, arylate, aryl hydride or an organic base such as M-isopropyl amide or M-hexamethyldisilylazide, wherein M is a metal Group 1 of the periodic table, in order to obtain the corresponding covalently anchored N[(perfluoroalkyl)sulfonyl]⁻ perfluoroalkane sulfonimide salts 1i', 1j', 1k', 2j' and 2k'; 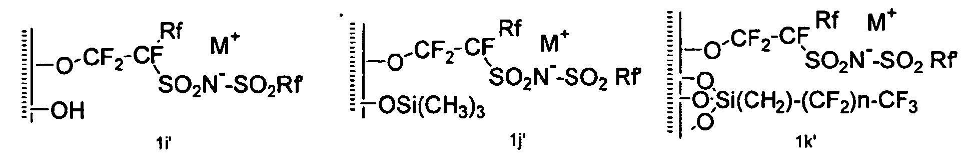 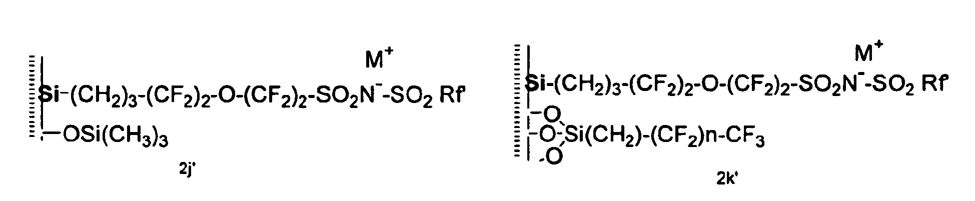
f) reacting the N[(perfluoroalkyl)sulfonyl]-perfluoroalkane sulfonimide salts-anchored supports of step e) with an organo-cation Cat⁺, in order to obtain respectively compounds 1l, 1m, 1n, 2m and 2n 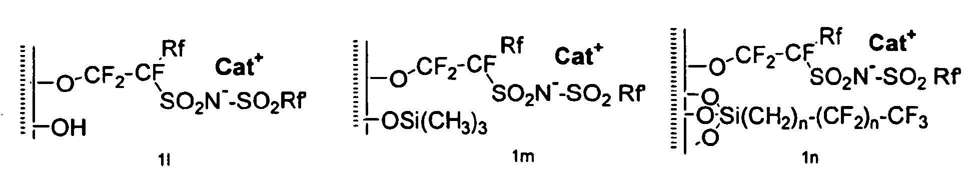 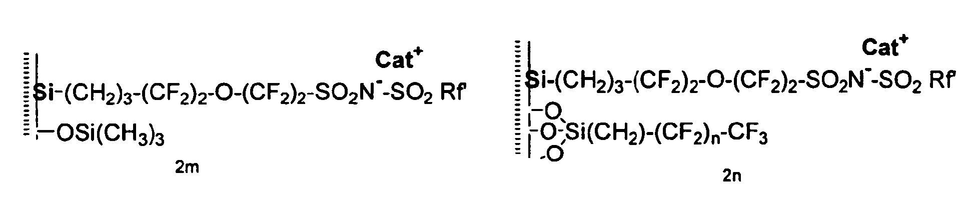 wherein Cat is trityl Ph₃CX or ammonium halide R₁R₂R₃R₄N⁺X⁻, wherein X is halogen, Ph is phenyl group, and R₁, R₂, R₃ and R₄ are each independently selected from hydrocarbons having from 1 to 6 carbon atoms.

2. The method of claim 1 wherein N[(perfluoroalkyl)sulfonyl] perfluoroalkane sulfonimide compounds 1i, 1j, 1k, 2j and 2k are synthesised in one step by reacting the perfluorosulfonyl chloride group-containing solids 1c, 1d, 1e, 2d and 2e of step b) with perflorosulfonamide Rf'SO₂NH₂ in presence of a solution of organic base.

3. The method of claim 1 wherein both anionic and cationic parts are anchored onto the support, by adding to compound 1i' the compound 1,3-diakyl-imidazolium bearing trialkoxysilyl groups (imid), said trialkoxysilyl group reacting with the residual hydroxyl groups of the mineral oxide surface to anchor the cationic part on the support 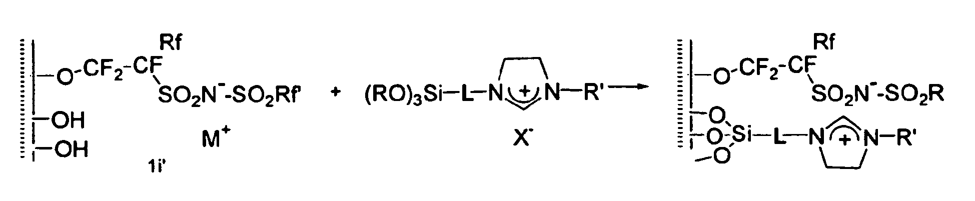 wherein linker group L is alkane or aryl group, R' is alkyl or aryl group, functionalised or not, and Rf and R'f are the same or different and are perfluoroalkyl groups CF₃(CF₂)ₙ- or (CF₃)₂CF(CF₂)ₙ- and wherein n is zero or an integer from 1 to 8.

4. The method of any one claims 1 to 3 wherein Rf and Rf are the same.

5. The method of claim 4 wherein Rf and Rf are both CF₃.

6. The method of claim 1 wherein M is Li.

7. The method of claim 1 wherein Cat is trityl or ammonium halide.

8. The supported ionic liquids and activating supports obtainable by the method of any one of claims 1 to 7.

9. A method for preparing an active catalyst system that comprises the steps of:
a) providing the supported ionic liquid or activating support of claim 8;
b) impregnating a metallocene or a post-metallocene catalyst component onto the activating support or supported ionic liquid;
c) optionally adding a scavenger;
d) retrieving an active catalyst system.

10. An active catalyst systems obtainable by the method of claim 9.

11. A method for oligomerising or homo- or co-polymerising ethylene and alpha-olefins that comprises the steps of:
a) injecting the active catalyst system of claim 10 into the reactor;
b) injecting the monomer and optional comonomer simultaneously with or after the catalyst system;
c) optionally injecting a scavenger;
d) maintaining under polymerisation conditions;
e) retrieving a polymer or oligomer.

12. The method of claim 11 wherein the monomer is selected from ethylene or propylene and the comonomer is selected from ethylene, propylene or 1-hexene.

## Patentansprüche

1. Verfahren zum Herstellen von trägergebundenen ionischen Flüssigkeiten oder Aktivierungsträgern durch kovalente Verankerung von nicht-koordinativen perfluorierten Anionen auf einem Mineraloxidträger, das die folgenden Schritte umfasst:
a) Umsetzen einer trägergebundenen Ausgangssulfonsäure 1 oder 2 entweder mit nicht-nukleophilem Trimethylsilan in Schema (a) oder mit Perfluoralkan-2-ethyltrialkoxysilan in Schema b), um die Sulfongruppenumgebung hydrophob zu machen 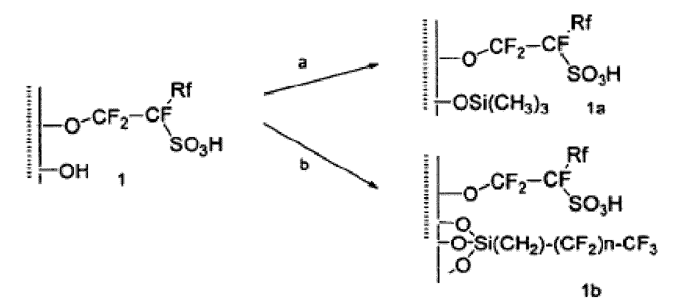 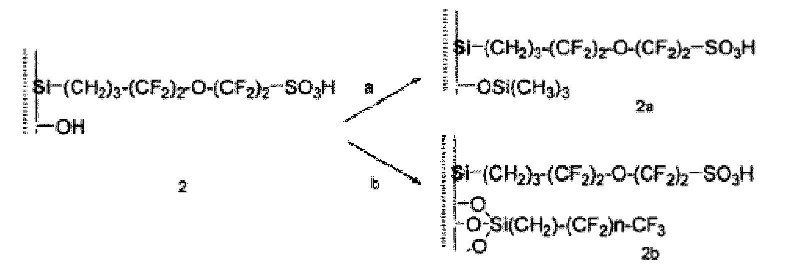 wobei Rf eine Perfluoralkylgruppe CF₃(CF₂)ₙ- oder (CF₃)₂CF(CF₂)n- ist, und wobei n 0 oder eine ganze Zahl von 1 bis 8 ist,
b) Umsetzen der Verbindungen 1, 1a, 1b, 2a und 2b mit Thionylchlorid, um trägergebundene Perfluorsulfonchloridprodukte 1c, 1d, 1e, 2d bzw. 2e zu produzieren, 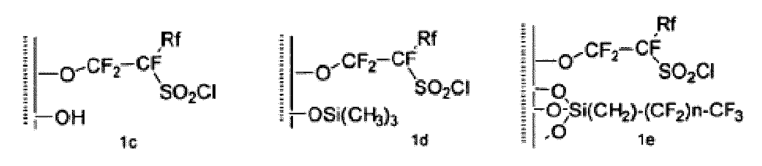 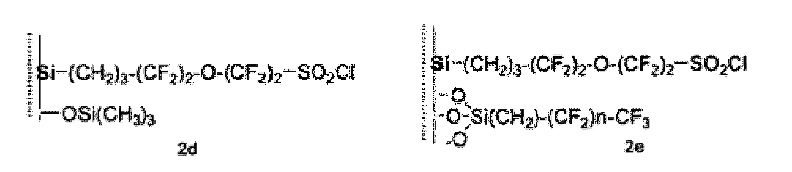
c) Umsetzen der Perfluorsulfonylchloridgruppen-haltigen Feststoffe 1c, 1d, 1e, 2d und 2e mit Ammoniak, um Perfluorsulfonamidverbindungen 1f, 1g, 1h, 2g und 2h herzustellen 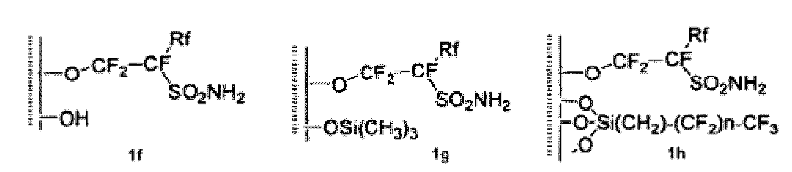 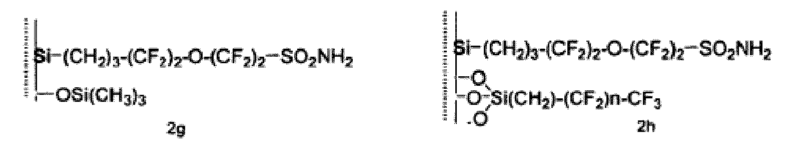
d) Umsetzen der Perfluorsulfonamidverbindungen 1f, 1g, 1h, 2g und 2h mit Perfluoralkansulfonylchlorid Rf'SO₂Cl in der Gegenwart einer organischen Base, um das kovalent verankerte N[(Perfluoralkyl)sulfonyl]perfluoralkansulfonamid 1i, 1j, 1k, 2j bzw. 2k herzustellen 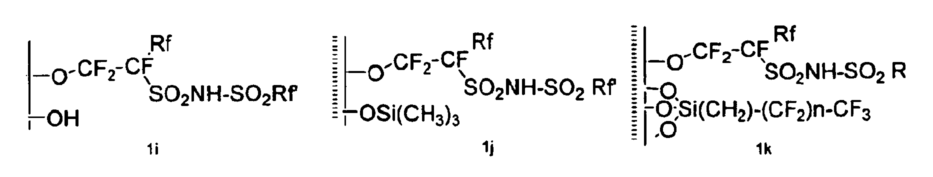 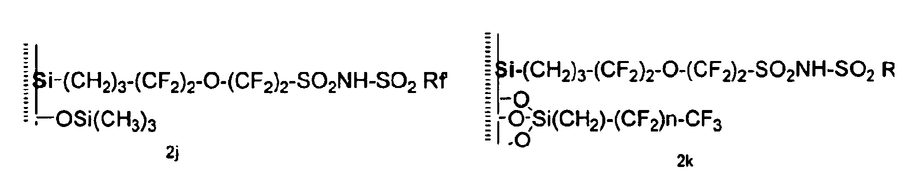 wobei Rf eine Perfluoralkylgruppe CF₂(CF₂)ₙ₋ oder (CF₃)₂CF(CF₂)ₙ₋ ist, wobei n 0 oder eine ganze Zahl von 1 bis 8 ist, und wobei Rf gleich wie Rf ist oder unterschiedlich davon,
e) Umsetzen des verankerten N[(Perfluoralkyl)sulfonyl]perfluoralkansulfonamids 1i, 1j, 1k, 2j und 2k mit einem Methallhydroxid MOH oder einem metallischen organischen Derivat wie Alkylat, Arylat, Arylhydrid oder einer organischen Base wie M-Isopropylamid oder M-Hexamethyldisilylazid, wobei M eine Metallgruppe 1 des Periodensystems ist, um die entsprechenden kovalent verankerten N[(Perfluoralkyl)sulfonyl]⁻ perfluoralkansulfonimidsalze 1i', 1j', 1k', 2j' und 2k' zu erhalten; 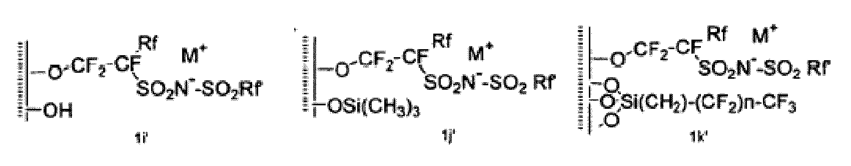 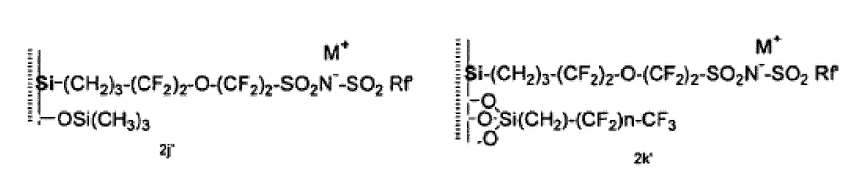
f) Umsetzen der N[(Perfluoralkyl)sulfonyl]⁻perfluoralkansulfonimidsalzverankerten Träger von Schritt e) mit einem Organokation Cat⁺, um Verbindungen 11, 1m, 1n, 2m bzw. 2n zu erhalten 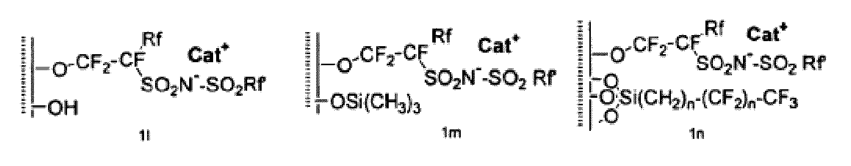 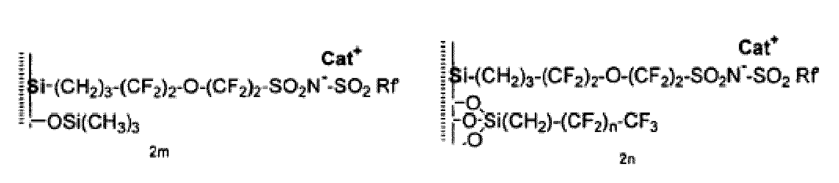 wobei Cat Trityl-Ph₃CX oder Ammoniumhalid R₁R₂R₃R₄N⁺X⁻ ist, wobei X Halogen ist, Ph eine Phenylgruppe ist, und R₁, R₂, R₃ und R₄ jeweils unabhängig aus Kohlenwasserstoffen mit 1 bis 6 Kohlenstoffatomen ausgewählt sind.

2. Verfahren nach Anspruch 1, wobei die N[(Perfluoralkyl)sulfonyl]perfluoralkansulfonimidverbindungen 1i, 1j, 1k, 2j und 2k in einem Schritt durch Umsetzen der Perfluorsulfonylchloridgruppen-haltigen Feststoffe 1c, 1d, 1e, 2d und 2e von Schritt b) mit Perfluorsulfonamid Rf'SO₂NH₂ in der Gegenwart einer Lösung aus organischer Base synthetisiert werden.

3. Verfahren nach Anspruch 1, wobei sowohl anionische als auch kationische Teile am Träger durch Hinzufügen der 1,3-Dialkylimidazolium-tragenden Trialkoxysilylgruppen (imid) zur Verbindung 1i' verankert werden, wobei die Trialkoxysilylgruppe mit den Resthydroxylgruppen der Mineraloxidoberfläche reagiert, um den kationischen Teil am Träger zu verankern, 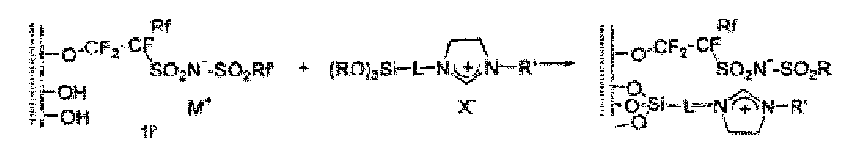 wobei Linkergruppe L Alkan- oder Arylgruppe ist, R' eine Alkyl- oder Arylgruppe ist, funktionalisiert oder nicht, und Rf und R'f gleich oder unterschiedlich und Perfluoralkylgruppen CF₃(CF₂)ₙ₋ oder (CF₃)₂CF(CF₂)ₙ- sind, und wobei n 0 oder eine ganze Zahl von 1 bis 8 ist.

4. Verfahren nach einem der Ansprüche 1 bis 3, wobei Rfund Rf' gleich sind.

5. Verfahren nach Anspruch 4, wobei Rf und Rf jeweils CF₃ sind.

6. Verfahren nach Anspruch 1, wobei M Li ist.

7. Verfahren nach Anspruch 1, wobei Cat Trityl oder Ammoniumhalid ist.

8. Trägergebundene ionische Flüssigkeiten und Aktivierungsträger, die mithilfe des Verfahrens nach einem der Ansprüche 1 bis 7 erhältlich sind.

9. Verfahren zum Herstellen eines aktiven Katalysatorsystems, das die Schritte umfasst:
a) Bereitstellen der trägergebundenen ionischen Flüssigkeit oder Aktivierungsträgers nach Anspruch 8,
b) Imprägnieren einer Metallocen- oder einer Post-Metallocenkatalysatorkomponente auf den Aktivierungsträger oder die trägergebundene ionische Flüssigkeit;
c) optional Hinzufügen eines Scavenger;
d) Gewinnen eines aktiven Katalysatorsystems.

10. Aktives Katalysatorsystem, das mithilfe des Verfahrens nach Anspruch 9 erhältlich ist.

11. Verfahren zum Oligomerisieren oder Homo- oder Copolymerisieren von Ethylen und alpha-Olefinen, das die Schritte umfasst:
a) Injizieren des aktiven Katalysatorsystems nach Anspruch 10 in den Reaktor;
b) Injizieren des Monomers und des optionalen Comonomers gleichzeitig mit dem Katalysatorsystem oder danach;
c) optional Injizieren eines Scavenger;
d) Halten unter Polymerisationsbedingungen;
e) Gewinnen eines Polymers oder Oligomers.

12. Verfahren nach Anspruch 11, wobei das Monomer aus Ethylen oder Propylen ausgewählt ist, und wobei das Comonomer aus Ethylen, Propylen oder 1-Hexen ausgewählt ist.

## Revendications

1. Procédé pour préparer des liquides ioniques supportés ou des supports d'activation par ancrage covalent d'anions perfluorés non-coordonnants sur un support en oxyde minéral, qui comprend les étapes consistant à :
a) faire réagir l'acide sulfonique supporté de départ 1 ou 2 soit avec un triméthylsilane non nucléophile dans le schéma (a) soit avec un perfluoroalcane-2-éthyltrialcoxy-silane dans le schéma (b) afin de rendre hydrophobe l'environnement du groupe sulfonique : 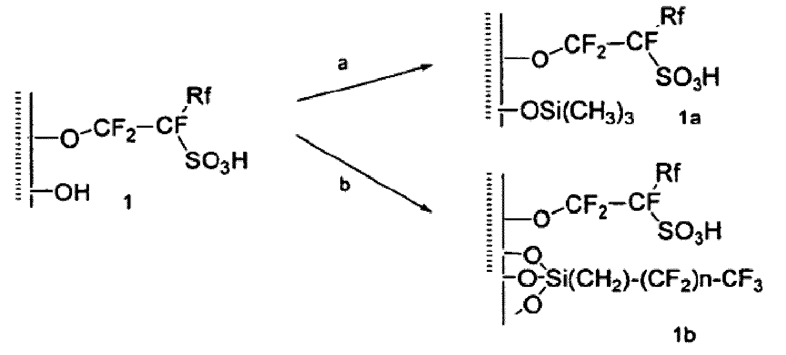 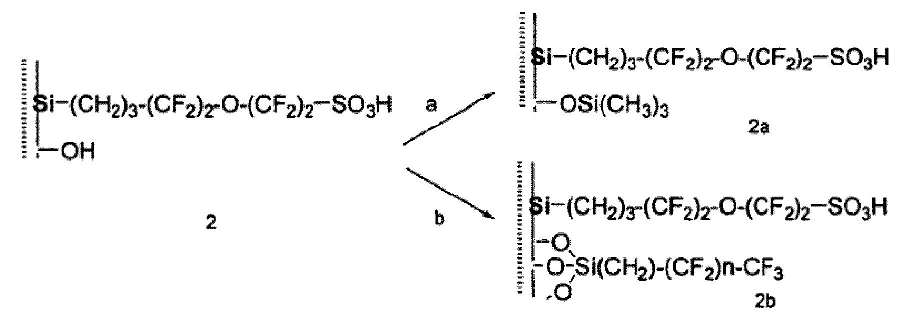 où Rf est un groupe perfluoroalkyle CF₃(CF₂)ₙ- ou (CF₃)₂CF(CF₂)ₙ- et où n vaut 0 ou est un entier de 1 à 8 ;
b) faire réagir les composés 1, 1 a, 1b, 2a et 2b avec du chlorure de thionyle pour produire les produits chlorures perfluorosulfoniques supportés respectivement 1 c, 1 d, 1 e, 2d et 2e : 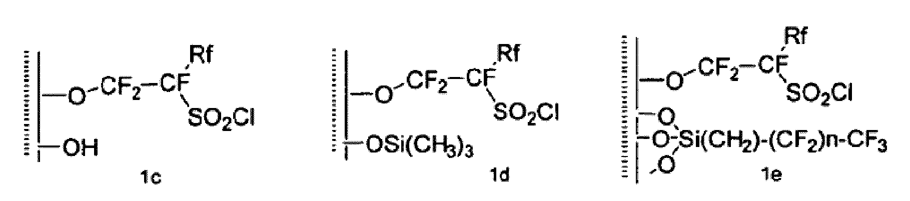 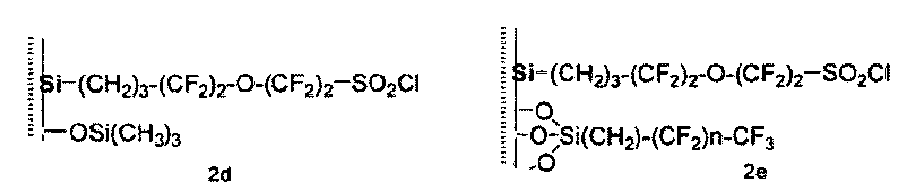
c) faire réagir les solides contenant un groupe chlorure de perfluorosulfonyle 1 c, 1 d, 1 e, 2d et 2e avec de l'ammoniac pour préparer les composés perfluorosulfonamides 1f, 1g, 1 h, 2g et 2h : 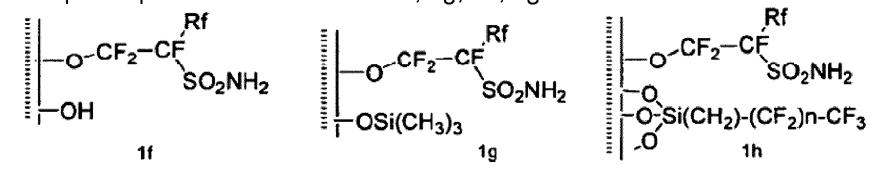 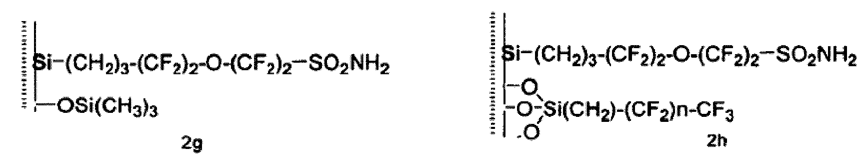
d) faire réagir les composés perfluorosulfonamides 1f, 1g, 1h, 2g et 2h avec un chlorure de perfluoroalcanesulfonyle Rf'SO₂Cl en présence d'une base organique pour préparer respectivement les N-[(perfluoroalkyl)sulfonyl]perfluoroalcanesulfonamides ancrés de manière covalente 1 i, 1j, 1 k, 2j et 2k : 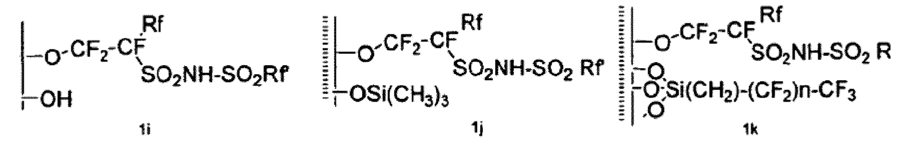 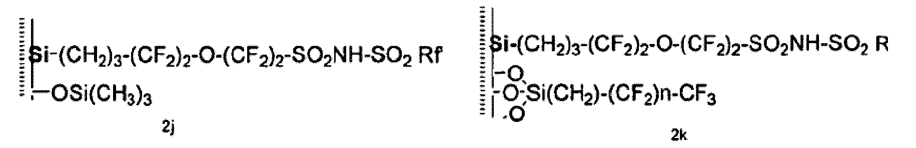 où Rf est un groupe perfluoroalkyle CF₃(CF₂)ₙ- ou (CF₃)₂CF(CF₂)ₙ- où n vaut 0 ou est un entier de 1 à 8 et où Rf est identique à ou différent de Rf ;
e) faire réagir les N-[(perfluoroalkyl)sulfonyl]-perfluoroalcanesulfonamides ancrés 1i, 1j, 1 k, 2j et 2k avec un hydroxyde métallique MOH ou un dérivé organométallique tel qu'un alkylate, arylate, hydrure d'aryle ou une base organique telle qu'un M-isopropylamide ou un azoture de M-hexaméthyldisilyle, où M est un métal du Groupe 1 du Tableau Périodique, afin d'obtenir les sels de N-[(perfluoroalkyl)sulfonyl]perfluoroalcanesulfonimide ancrés de manière covalente correspondants 1i', 1j', 1 k', 2j' et 2k' : 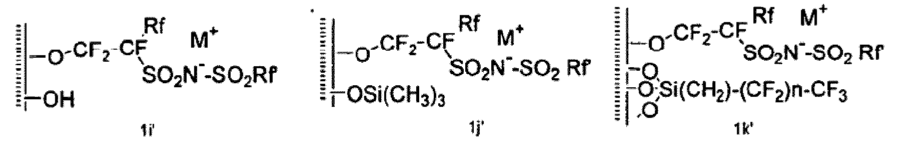 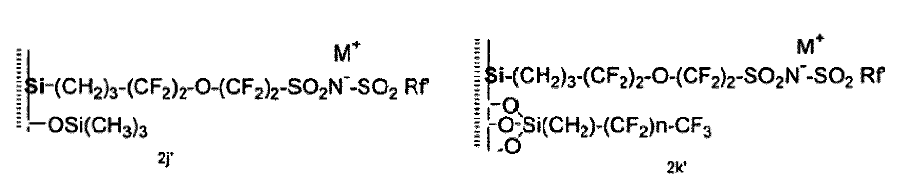
f) faire réagir les supports ancrés de sels de N-[(perfluoroalkyl)sulfonyl]perfluoroalcanesulfonimide de l'étape e) avec un cation organique Cat⁺ afin d'obtenir respectivement les composés 1l, 1m, 1n, 2m et 2n : 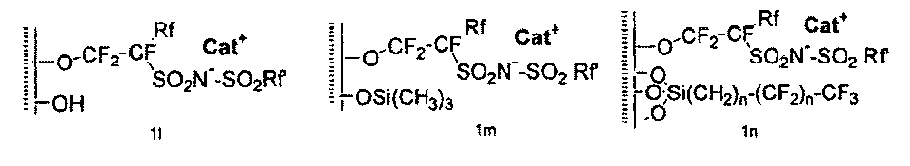 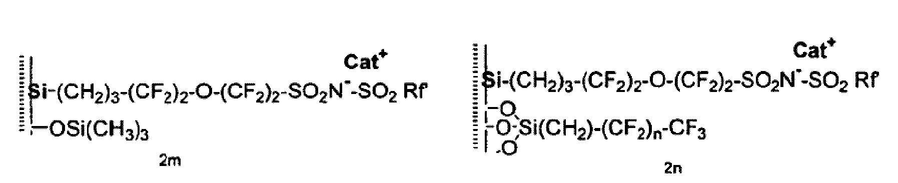 où Cat est un trityle Ph₃CX ou un halogénure d'ammonium R₁R₂R₃R₄N⁺X⁻ où X est un halogène, Ph est un groupe phényle, et chacun de R₁, R₂, R₃ et R₄ est indépendamment choisi parmi les hydrocarbures ayant de 1 à 6 atomes de carbone.

2. Procédé selon la revendication 1, dans lequel les composés N-[(perfluoroalkyl)sulfonyl]perfluoro-alcanesulfonamides 1i, 1j, 1 k, 2j et 2k sont synthétisés en une seule étape par réaction des solides contenant un groupe chlorure de perfluorosulfonyle 1 c, 1 d, 1 e, 2d et 2e de l'étape b) avec un perfluorosulfonamide RfSO₂NH₂ en présence d'une solution de base organique.

3. Procédé selon la revendication 1, dans lequel les parties tant anionique que cationique sont ancrées sur le support, par addition au composé 1i' d'un composé 1,3-dialkyl-imidazolium portant des groupes trialcoxysilyle (imide), ledit groupe trialcoxysilyle réagissant avec les groupes hydroxyle résiduels de la surface de l'oxyde minéral pour ancrer la partie cationique sur le support : 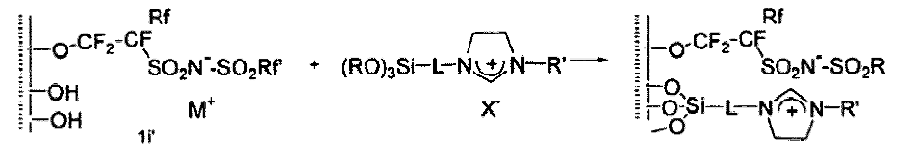 où le groupe lieur L est un groupe alcane ou aryle, R' est un groupe alkyle ou aryle, fonctionnalisé ou non, et Rf et R'f sont identiques ou différents et sont des groupes perfluoroalkyle CF₃(CF₂)ₙ- ou (CF₃)₂CF(CF₂)ₙ- et où n vaut zéro ou est un entier de 1 à 8.

4. Procédé selon l'une quelconque des revendications 1 à 3, dans lequel Rf et Rf sont identiques.

5. Procédé selon la revendication 4, dans lequel Rf et Rf sont tous deux CF₃.

6. Procédé selon la revendication 1, dans lequel M est Li.

7. Procédé selon la revendication 1, dans lequel Cat est un trityle ou un halogénure d'ammonium.

8. Liquides ioniques supportés et supports d'activation pouvant être obtenus par le procédé de l'une quelconque des revendications 1 à 7.

9. Procédé pour préparer un système catalyseur actif, comprenant les étapes consistant à :
a) disposer du liquide ionique supporté ou du support d'activation de la revendication 8 ;
b) imprégner un composant catalyseur métallocène ou post-métallocène sur le support d'activation ou le liquide ionique supporté ;
c) éventuellement ajouter un piégeur ;
d) récupérer un système catalyseur actif.

10. Systèmes catalyseurs actifs pouvant être obtenus par le procédé de la revendication 9.

11. Procédé pour oligomériser ou homo- ou co-polymériser de l'éthylène et des α-oléfines, qui comprend les étapes consistant à :
a) injecter le système catalyseur actif de la revendication 10 dans le réacteur ;
b) injecter le monomère et le comonomère facultatif en même temps que ou après le système catalyseur ;
c) éventuellement injecter un piégeur ;
d) maintenir dans des conditions de polymérisation ;
e) récupérer un polymère ou oligomère.

12. Procédé selon la revendication 11, dans lequel le monomère est choisi parmi l'éthylène et le propylène, et le comonomère est choisi parmi l'éthylène, le propylène et le 1-hexène.
